# EUROPÄISCHE PATENTSCHRIFT

(11) **EP 1 657 230 B1**
(45) Veröffentlichungstag und Bekanntmachung des Hinweises auf die Patenterteilung: **25.03.2009**
(21) Anmeldenummer: 05024054.8
(22) Anmeldetag: 04.11.2005
(51) Int. Cl.: C07C 51/235, C07C 53/126

(54) **Verfahren zur Herstellung von aliphatischen geradkettigen und ß-alkylverzweigten Carbonsäuren**
Process for the preparation of aliphatic linear and ß-alkyl-branched carboxylic acids
Procédé de préparation d'acides carboxyliques aliphatiques linéaires et ß-alkyles ramifiés

(30) Priorität: 16.11.2004 DE 102004055252
(43) Veröffentlichungstag der Anmeldung: 17.05.2006
(73) Patentinhaber: OXEA Deutschland GmbH, 46147 Oberhausen (DE)
(72) Erfinder: Springer, Helmut, 46539 Dinslaken (DE); Winsberg, Frank, 47169 Duisburg (DE)

(56) Entgegenhaltungen:
- DE-C1- 10 010 771
- GB-A- 856 962

## Beschreibung

Die vorliegende Erfindung betrifft ein neues, katalytisches Verfahren zur Herstellung von aliphatischen geradkettigen und β-alkylverzweigten Carbonsäuren aus Aldehyden durch Oxidation mit Sauerstoff oder Sauerstoff enthaltenden Gasen.

Aldehyde werden in großem Umfang als Ausgangsstoffe für die Herstellung von Carbonsäuren eingesetzt. Die Vorzugsstellung für diese Verwendung verdanken Aldehyde ihrer hohen Verfügbarkeit nach einer Reihe, auch industriell genutzter Prozesse. Überdies läßt sich die Carbonylgruppe der Aldehyde leicht in die Carboxylgruppe umwandeln. Im Rahmen technisch ausgeübter Verfahren erfolgt die Umsetzung von Aldehyden zu Carbonsäuren vorwiegend in Anwesenheit von Katalysatoren. Als Katalysatoren kommen vorwiegend Salze von Übergangsmetallen in Betracht, insbesondere Salze des Kobalts und des Mangans sowie des Chroms, Eisens, Kupfers, Nickels, Silbers und Vanadiums. Dennoch ist die Carbonsäurebildung aus Aldehyden, selbst bei Einhaltung optimaler Temperaturbedingungen, häufig mit Neben- und Abbaureaktionen verbunden.

J. Prakt. Chem. Bd. 14 (1961), 71-83 beschreibt die Oxidation von Isononanal in Gegenwart von Kobaltacetat oder Mangannaphthenat. In Gegenwart des Mangan enthaltenden Katalysators beträgt bei einer Reaktionstemperatur von 60°C die Ausbeute an Isononansäure nur ca. 70%.

Nach dem in der DE-OS 30 29 700 beschriebenen Verfahren werden zur Herstellung von aliphatischen Monocarbonsäuren mit 6 bis 9 Kohlenstoffatomen die entsprechenden Aldehyde mit Sauerstoff in reiner Form oder mit Luft oxidiert. Als Katalysator wirkt eine Kombination von Mangan- und Kupferverbindungen, die in der Säure löslich sind. Die Metalle liegen in einer Menge von je etwa 10 bis etwa 2000 ppm, vorzugsweise 200 bis 600 ppm Mangan und Kupfer, bezogen auf das Gewicht des flüssigen Reaktionsgemischs, vor. Das Molverhältnis von Mangan zu Kupfer beträgt 5:1 bis 0,5:1. Die Umsetzung der Ausgangsstoffe erfolgt in flüssiger Phase bei Temperaturen von etwa 50 bis 80°C und Drücken im Bereich von etwa 1,4 bis 10,3 bar. Als Hauptschwierigkeit dieses Verfahrens wird in der Prozessbeschreibung die Anwesenheit von Kupfer- und auch Manganverbindungen im Reaktionsprodukt, d.h. in der Carbonsäure, geschildert. Zur Entfernung der Metalle sind aufwendige Reinigungsmaßnahmen erforderlich, beispielsweise ihre Ausfällung mit wässriger Oxalsäure.

Das in der US-Patentschrift 4 487 720 offenbarte Verfahren zur Herstellung von C₅- bis C₉-Monocarbonsäuren durch Oxidation von Aldehyden der gleichen Kohlenstoffatom-Zahl mit reinem Sauerstoff oder mit Luft arbeitet ebenfalls mit Kupfer- und Manganverbindungen als Katalysatoren. Die Gesamtmenge der Metalle erstreckt sich über einen Bereich von 10 bis 200 ppm, bezogen auf das Gesamtgewicht der aus Aldehyd, Säure und Katalysator bestehenden Lösung. Mangan und Kupfer werden in einem Molverhältnis von etwa 3:1 bis etwa 1:1 eingesetzt. Als Nachteil dieser Arbeitsweise wird die Bildung von Kupferfilmen beschrieben, die bei der destillativen Reinigung der Säure auftreten und mechanische Schäden in der Destillationsapparatur zur Folge haben. Um dieses Problem zu vermeiden wird empfohlen, die Destillation in Gegenwart von Sauerstoff durchzuführen.

Gegenstand der bekanntgemachten deutschen Patentanmeldung 26 04 545 ist die Herstellung von Alkylcarbonsäuren der allgemeinen Formel CₙH₂ₙ₊₁COOH, in der n einen Wert von 2 bis 18 bedeutet, durch Hydroformylierung, auch bekannt unter der Bezeichnung Oxosynthese, eines Olefins der allgemeinen Formel CₙH₂ₙ und durch unmittelbare Oxidation des bei der Hydroformylierung anfallenden Reaktionsgemisches. Unmittelbar heißt in diesem Zusammenhang, daß die vorherige Aufarbeitung des Hydroformylierungsgemischs unterbleibt und die nachfolgende Oxidationsreaktion in Gegenwart von Rhodium erfolgt. Das bekannte Oxidationsverfahren dient insbesondere zur Herstellung von Gemischen isomerer C₉-C₁₆-Fettsäuren. Als Ausgangsolefine für die Oxosynthese kommen bevorzugt die Dimere und Trimere des Propens und der Butene in Betracht, darunter vor allem das dimere Isobuten (2,4,4-Trimethylpenten-1). Beide Einzelumsetzungen des zweistufigen Verfahrens, d.h. sowohl die Hydroformylierung als auch die Oxidation, werden durch Rhodium in Form seiner Verbindungen katalysiert. Maßgebend für die Rhodium-Konzentration in dem der Oxidation unterworfenen Reaktionsgemisch ist daher der relativ hohe Rhodiumanteil im Hydroformylierungsprodukt. Um die Wirtschaftlichkeit des Gesamtprozesses sicherzustellen ist es erforderlich, das Edelmetall durch geeignete Maßnahmen möglichst vollständig aus dem Endprodukt des Prozesses, der Carbonsäure, wiederzugewinnen. Überdies ist nicht auszuschließen, dass durch Rhodium in der vorliegenden Konzentration während des Oxidationsvorganges unerwünschte Nebenreaktionen begünstigt werden, denn die Carbonsäure-Ausbeute ist, wie die Beispiele zeigen, für eine technische Nutzung des Verfahrens unzureichend.

LARKIN berichtet in J.Org.Chem. 1990, 55, S. 1563 ff., dass die Gegenwart von Katalysatoren bei der kommerziell ausgeführten Oxidation von Aldehyden zu Carbonsäuren deshalb für notwendig erachtet wird, weil im Reaktionsgemisch Spuren von Metallsalzen enthalten sind, die Nebenreaktionen katalysieren können. Die Bildung der Metallsalze geht auf die Korrosion metallischer Anlagenteile zurück. Aufgabe der Katalysatoren ist es, die Wirkung der Korrosionsprodukte zu überkompensieren.

Auch in Ullmanns Encyklopädie der technischen Chemie, 4. Auflage 1975, Band 9, wird mehrfach auf den negativen Einfluss metallischer Verunreinigungen in den zur Oxidation eingesetzten Ausgangsaldehyden hingewiesen. So führen z.B. im Butyraldehyd gelöste Eisen- und Kobaltsalze bei dessen Oxidation zu Buttersäure zu einem erhöhten Anfall von Nebenprodukten (1.c., Seite 142, linke Spalte) und bei der Oxidation von 2-Ethylhexanal zu 2-Ethylhexansäure beschleunigen Schwermetallionen die Decarbonylierung des Ausgangsaldehyds zu Heptan (1.c., Seite 144, linke Spalte).

Es gibt Hinweise im Stand der Technik, nach denen die Wirkung von Katalysatorzusätzen von der Struktur des für die Oxidation eingesetzten Aldehyds abhängt. So ist beispielsweise aus DE 950 007 bekannt, dass die Oxidation von in α-Stellung verzweigten Aldehyden den Zusatz geringer Mengen von carbonsauren Alkalisalzen erfordert, um die gewünschten Carbonsäuren in hoher Ausbeute bei gleichzeitig hoher Reinheit zu erhalten.

Nach der Lehre der offengelegten japanischen Patentanmeldung 53-105413 oxidiert man α-verzweigte aliphatische Aldehyde mit Sauerstoff in Gegenwart von Lithium- oder Erdalkalimetallverbindungen, die in Mengen von 0,01 bis 10 Gew.-% (bezogen auf das gesamte Reaktionssystem) eingesetzt werden, um α-verzweigte aliphatische Carbonsäuren herzustellen.

Kennzeichnend für die in der französischen Patentanmeldung 2 769 624 beschriebene Arbeitsweise ist die Einhaltung niedriger Reaktionstemperaturen, nämlich Temperaturen zwischen 0 und 25°C. Das Verfahren erfordert ebenfalls die Gegenwart von Alkalimetall- bzw. Erdalkalimetallverbindungen als Hilfsstoffe. Es bleibt offen, welche speziellen Wirkungen diese Verbindungen entfalten, d.h. ob sie, wie bekannt, lediglich die Selektivität der Umsetzung verbessern oder aber bei den gewählten niedrigen Temperaturen, möglicherweise auch die Reaktionsgeschwindigkeit erhöhen.

Bei der Oxidation von α-verzweigten Aldehyden, bei denen das dem Carbonylkohlenstoffatom benachbarte Kohlenstoffatom die Verzweigung trägt, empfiehlt somit der Stand der Technik den Zusatz geringer Mengen an Alkalimetallcarboxylaten zur Selektivitätsverbesserung. Ein derartiger Zusatz ist jedoch aufgrund ihrer inhibierenden Wirkung mit einer Verlängerung der Reaktionszeit verbunden. Unter den α-verzweigten Aldehyden besitzt besonders 2-Ethylhexanal, das in großen Mengen zur 2-Ethylhexansäure weiterverarbeitet wird, eine wirtschaftliche Bedeutung.

Die Oxidation von Aldehyden, die in β-Position, das heißt an dem, bezogen auf das Carbonylkohlenstoffatom, übernächsten Kohlenstoffatom die Verzweigung tragen, kann ebenfalls unter Katalysatorzusatz erfolgen. Einen wirtschaftlich bedeutenden Aldehyd mit einem hohen Anteil an β-alkylverzweigten Verbindungen erhält man durch Hydroformylierung von technisch verfügbarem Diisobuten (2,4,4-Trimethylpenten-1). Die Oxidation in Gegenwart von Rhodium nach DE-A1-26 04 545 liefert ein Gemisch isomerer C₉-Fettsäuren mit einem hohen Anteil an 3,5,5-Trimethylhexansäure, häufig auch als Isononansäure bezeichnet. Die Oxidation von β-alkylverzweigten Aldehyden, beispielsweise von Isovaleraldehyd, in Gegenwart von Alkali- oder Erdalkalicarboxylaten ist aus DE-A1-732 720 bekannt.

Lineare Aldehyde lassen sich nach der Lehre gemäß DE-C1-100 10 771 in Gegenwart von Übergangsmetallen oder deren Verbindungen in die entsprechenden Carbonsäuren überführen.

Die Patentschrift DE-C1-100 10 771 offenbart ebenfalls den Einsatz eines Gemisches aus Alkalisalzen und Übergangsmetallen bei der Oxidation von 2-Methylbutanal als α-verzweigten Aldehyd.

GB 856 962 A behandelt ein Flüssigphasenverfahren zur Oxidation von gesättigten aliphatischen Monoaldehyden zu den entsprechenden Carbonsäuren in Gegenwart von Kobalt- und/oder Mangansalzen, wobei zusätzlich noch Verbindungen des Lithiums, Natriums, Rubidiums, Caesiums, Calciums, Strontiums oder Bariums zugegen sind und wenigstens eine dieser Verbindungen in ionischer Form vorliegt.

Die bekannten katalytischen Verfahren zur Oxidation von aliphatischen geradkettigen Aldehyden und von β-alkylverzweigten Aldehyden zu den entsprechenden Carbonsäuren erfüllen noch nicht im vollen Umfang die technischen und wirtschaftlichen Erfordernisse, die an moderne, industriell genutzte Prozesse gestellt werden. Die Anwendung von Übergangsmetallkatalysatoren hat oftmals das Auftreten unerwünschter Nebenreaktionen zur Folge, die die Selektivität mindern, so dass trotz eines hohen Umsatzes die Ausbeute zu den gewünschten Carbonsäuren leidet. Der Zusatz von Alkali- und/oder Erdalkalimetallcarboxylaten verbessert zwar das Selektivitätsverhalten, wirkt sich jedoch nachteilig auf das Umsatzverhalten aus.

Es bestand daher die Aufgabe, ein Verfahren für die Oxidation von aliphatischen geradkettigen Aldehyden und von β-alkylverzweigten Aldehyden bereitzustellen, das die zuvor erwähnten Nachteile nicht aufweist und das bei hohen Aldehydumsätzen mit hoher Selektivität zu den gewünschten Carbonsäuren führt. Als Ergebnis wird die Gewinnung von geradkettigen Carbonsäuren und von β-alkylverzweigten Carbonsäuren aus den entsprechenden Aldehyden in hoher Ausbeute und Reinheit angestrebt.

Die Aufgabe wird gelöst durch ein Verfahren zur Herstellung von aliphatischen geradkettigen und von β-alkylverzweigten Carbonsäuren mit 5 bis 13 Kohlenstoffatomen durch Oxidation der entsprechenden Aldehyde mit Sauerstoff oder Sauerstoff enthaltenden Gasgemischen bei 20 bis 100°C. Es ist dadurch gekennzeichnet, dass die Oxidation der Aldehyde in Gegenwart von Kaliumcarboxylat in einer Menge berechnet als Kaliummetall, von 1 mmol bis 10 mmol je mol eingesetztem Aldehyd und in Gegenwart von 0,1 bis 5,0 ppm Eisen oder der entsprechenden Menge einer Eisenverbindung, bezogen auf eingesetzten Aldehyd, erfolgt.

Überraschenderweise gelingt es, in Gegenwart geringer Mengen an Kaliumcarboxylat und in Gegenwart geringer Mengen Eisen oder Eisenverbindungen, aliphatische geradkettige oder β-alkylverzweigte Aldehyde mit reinem Sauerstoff oder Sauerstoff enthaltenden Gasgemischen mit hoher Selektivität und gleichzeitig hohem Umsatz zu den entsprechenden Carbonsäuren umzusetzen.

Wesentliches Merkmal des neuen Verfahrens ist das gleichzeitige Vorliegen von Kaliumcarboxylat und Eisen als katalytisch wirksames Metall in dem Oxidationsgemisch. Es hat sich überraschenderweise gezeigt, dass bei gleichzeitigem Vorliegen von Kaliumcarboxylat und Eisen als katalytisch wirksames Metall in dem Oxidationsgemisch die Selektivität in der Oxidation von geradkettigen aliphatischen Aldehyden bei hohem Umsatz noch weiter verbessert werden kann, so dass insgesamt eine höhere Ausbeute an geradkettigen, aliphatischen Carbonsäuren beobachtet wird gegenüber einer Arbeitsweise, bei der die Oxidation geradkettiger aliphatischer Aldehyde nur unter Metallzusatz gemäß DE-C1-100 10 771 erfolgt. Auch lässt sich bei der Oxidation von β-alkylverzweigten Aldehyden der Aldehydumsatz und die Selektivität zu den gewünschten Carbonsäuren gegenüber der Lehre nach DE-C1-100 10 771 nochmals erhöhen, so dass die erfindungsgemäße Arbeitsweise zu einer deutlichen Ausbeutesteigerung bei der Oxidation von β-alkylverzweigten Aldehyden führt.

Je mol Aldehyd betragen die eingesetzten Mengen an Kaliumcarboxylat berechnet als Kaliummetall, 1 mmol bis zu 10 mmol. Zusätze in geringerer Menge ergeben keine Vorteile während bei Zusätzen von über 10 mmol Kaliumcarboxylat je mol Aldehyd, berechnet als Kaliummetall, die beobachteten Ergebnisse der Verfahrensvariante entsprechen, bei der ohne Zusatz von Kaliumcarboxylat gearbeitet wird.

Die Gesamtmenge an zugesetztem Kaliumcarboxylat, sollte einen maximalen Gesamtwert von 30 mmol Kaliummetall, bezogen auf 1 mol Aldehyd, nicht übersteigen.

Besonders hohe Ausbeuten erzielt man, wenn man je mol Aldehyd 1 bis 8 und insbesondere 1 bis 5 mmol Kaliumcarboxylat, berechnet als Kaliummetall, zusetzt.

Man setzt eine einheitliche Verbindung ein, beispielsweise Kalium-isononanoat oder Kaliumpentanoat.

Im Allgemeinen stellt man die Kaliumcarboxylat enthaltende Lösung durch Neutralisation einer wässrigen die Kaliumverbindung enthaltenden Lösung mit einem Überschuß an der jeweils gewünschten Carbonsäure her und setzt diese Lösung dem zu oxidierenden Aldehyd zu. Als Kaliumverbindungen eignen sich besonders die Hydroxide, Carbonate oder Hydrogencarbonate.

Es ist aber auch möglich, das Kaliumcarboxylat im Reaktionsgemisch zu erzeugen, indem man ihm Kaliumverbindungen zusetzt, die unter den Reaktionsbedingungen in das Carboxylat überführt werden. Beispielweise lassen sich Kaliumhydroxid, -carbonat, -hydrogencarbonat oder -oxid in dem erfindungsgemäßen Verfahren einsetzen. Ihr Zusatz kann entweder in fester Form oder als wässrige Lösung erfolgen.

Als Katalysator wird dem Oxidationsgemisch neben dem Kaliumcarboxylat erfindungsgemäß Eisen oder mindestens eine Eisenverbindung zugesetzt. Verwendet man Eisen in elementarer Form als Katalysatoren, so empfiehlt es sich, es in feiner Verteilung dem Reaktionsgemisch hinzuzufügen. Statt Eisen in elementarer Form können auch Eisenverbindungen als Katalysatoren Anwendung finden. Die Art der Verbindungen unterliegt dabei keiner Beschränkung. Sofern nicht besondere Gründe vorliegen, wird man jedoch solche Verbindungen bevorzugen, die im Reaktionsmedium von Anfang an löslich sind, um eine Verzögerung des Reaktionseintritts durch vorherige Bildung einer löslichen und damit besonders aktiven Metallverbindung zu vermeiden.

Zu dem katalytisch, bereits in sehr geringer Menge wirksamen Metall zählt Eisen. Als im Reaktionsgemisch lösliche Verbindungen verwendet man Salze, insbesondere Salze organischer Säuren, wobei Carboxylate der Säuren bevorzugt werden, die das Ergebnis der Oxidationsreaktion sind. Andere geeignete Verbindungen des erfindungsgemäß eingesetzten Eisens sind Komplexverbindungen, z.B. Acetylacetonate, Metallcarbonyle, Hydridometallcarbonyle, ferner Carbonylverbindungen, die neben Kohlenmonoxid und gegebenenfalls Wasserstoff noch weitere Liganden, z.B. durch organische Reste substituierte Phosphine wie Arylphosphine, Alkylphosphine, Arylalkylphosphine enthalten. Ein Beispiel für derartige Liganden ist das Triphenylphosphin.

Bei dem erfindungsgemäßen Verfahren ist ein maximales Gewichtsverhältnis zwischen Eisen und zu oxidierendem Aldehyd einzuhalten. Erfindungsgemäß ist die obere Grenze dieses Verhältnisses 5 ppm, d.h. 5 Gew.-Teile Eisen je 10⁶ Gew.-Teile Aldehyd. Es hat sich besonders bewährt, auf 10⁶ Gew.-Teile Aldehyd 0,2 bis 3 Gew.-Teile Eisen und vorzugsweise 0,5 bis 2 Gew.-Teile Eisen anzuwenden. Die vorstehend beschriebenen Verhältnisse zwischen Eisen und Aldehyd gelten auch bei Verwendung von Eisenverbindungen, d.h. die Menge der einzusetzenden Verbindung bemisst sich nach ihrem Eisengehalt.

Die angewandten Eisenmengen stellen eine, auch für technische Bedürfnisse, ausreichende Reaktionsgeschwindigkeit sicher. Sie geben jedoch nicht zu unerwünschten Nebenreaktionen Anlass, so dass die Aldehyde nahezu ausschließlich in die ihnen entsprechenden Carbonsäuren umgewandelt werden. Überdies sind die eingesetzten Eisenmengen so gering, dass sie weder unter dem Aspekt der Wirtschaftlichkeit des Verfahrens, noch im Hinblick auf die für die verschiedenen Anwendungsgebiete geforderte Reinheit der Carbonsäuren, aus dem Reaktionsprodukt wiedergewonnen bzw. entfernt werden müssen.

Das Verfahren der Erfindung wird in einem Temperaturbereich von 20 bis 100°C durchgeführt. Vorzugsweise arbeitet man zwischen 20 und 80°C, insbesondere zwischen 40 und 80°C. Die Temperaturführung, konstante oder variable Temperatur, kann den individuellen Erfordernissen des Ausgangsmaterials und den Reaktionsgegebenheiten angepaßt werden.

Die Umsetzung der Reaktionspartner erfolgt bevorzugt bei Atmosphärendruck. Die Anwendung erhöhten Drucks ist jedoch nicht ausgeschlossen. Üblicherweise arbeitet man in einem Bereich von Atmosphärendruck bis 1,0 MPa, vorzugsweise bei Atmosphärendruck bis 0,8 MPa.

Die zur Umwandlung von Aldehyden in Carbonsäuren nach dem erfindungsgemäßen Verfahren erforderliche Reaktionszeit hängt unter anderem ab von deren Reaktionstemperatur, der Art der Einsatzstoffe und dem Mengenverhältnis der Reaktanten zueinander. Normalerweise beträgt sie 30 Minuten bis 20 Stunden, insbesondere 2 bis 8 Stunden.

Im Mittelpunkt des neuen Prozesses steht die Oxidation von aliphatischen geradkettigen oder β-alkylverzweigten Aldehyden mit 5 bis 13 Kohlenstoffatomen. Unter β-alkylverzweigten Aldehyden sind auch solche Aldehyde zu verstehen, die neben der β-Alkylverzweigung weitere Seitengruppen an dem Kohlenstoffatomgerüst tragen. Die Herkunft der Aldehyde ist nicht auf bestimmte Herstellungsverfahren beschränkt. Aufgrund ihrer leichten Zugänglichkeit werden durch Oxosynthese, d.h. durch Reaktion von Olefinen mit 4 bis 12 Kohlenstoffatomen mit Kohlenmonoxid und Wasserstoff gewonnene Aldehyde bevorzugt. In diesem Zusammenhang ist es nicht entscheidend, welche spezielle Ausführungsform der Oxosynthese zur Gewinnung der Aldehyde diente, d.h. ob die Reaktion z.B. durch Kobalt oder durch Rhodium katalysiert wurde, ob man die Metalle allein oder zusammen mit Komplexbildnern einsetzte und der Katalysator im Reaktionsgemisch homogen gelöst war oder eine eigene, heterogene Phase bildete.

Besonders eignet sich das erfindungsgemäße Verfahren zur Herstellung von Isononansäure aus dem Reaktionsprodukt der mit Diisobutylen durchgeführten Oxosynthese. Das technisch verfügbare Reaktionsprodukt der Oxosynthese von Diisobutylen enthält als Hauptbestandteil 3,5,5- Trimethylhexanal sowie geringe Mengen an 3,4,4-, und 3,4,5-Trimethylhexanal. Darüber hinaus sind geringe Mengen an nicht in β-Position verzweigten Aldehyden, wie 2,5,5-Trimethylhexanal, 4,5,5-Trimethylhexanal und 6,6-Dimethylheptanal zugegen. Die Oxidation dieses technisch verfügbaren Gemisches isomerer Nonanale nach dem erfindungsgemäßen Verfahren führt bei einem hohen Umsatz gleichzeitig zu einer ausgezeichneten selektiven Bildung von Isononansäure.

Ebenso ist das erfindungsgemäße Verfahren für die Oxidation von n-Pentanal, n-Heptanal, n-Nonanal sowie Isovaleraldehyd, zu den entsprechenden Carbonsäuren in hervorragender Weise geeignet.

Als Oxidationsmittel verwendet man nach dem Verfahren der Erfindung molekularen Sauerstoff oder Gasgemische, die molekularen Sauerstoff enthalten. Weitere Bestandteile derartiger Gasgemische sind inerte Gase, z.B. Stickstoff, Edelgase und Kohlendioxid. Der Anteil der inerten Bestandteile des Sauerstoff enthaltenden Gasgemisches beträgt bis zu 90 Vol-%, insbesondere 30 bis 80 Vol-%. Die bevorzugten Oxidationsmittel sind Sauerstoff oder Luft.

Die Aldehyde können als solche oder gelöst in einem, unter den Reaktionsbedingungen inerten Lösungsmittel eingesetzt werden. Beispiele für geeignete Lösungsmittel sind Ketone wie Aceton, Ester, z.B. Ethylacetat, Kohlenwasserstoffe, z.B. Toluol und Nitrokohlenwasserstoffe wie Nitrobenzol. Die Konzentration des Aldehyds wird durch seine Löslichkeit in dem Lösungsmittel begrenzt.

Das erfindungsgemäße Verfahren kann absatzweise oder kontinuierlich durchgeführt werden. Eine Rückführung nicht umgesetzter Reaktionsteilnehmer ist in beiden Fällen möglich.

Aus dem nach der Oxidation anfallenden Rohsäuregemisch wird mittels Destillation unter üblichen Bedingungen die reine Carbonsäure gewonnen. Der das Kaliumcarboxylat und Eisen enthaltende Destillationsrückstand wird abgetrennt und kann dem Einsatzaldehyd, gegebenenfalls nach Zugabe von frischem Kaliumcarboxylat oder Kaliumverbindungen, die unter den Reaktionsbedingungen in das Carboxylat übergehen, sowie von Eisen als katalytischem Metall wieder zugeführt werden.

Nach einer bewährten Ausführungsform des erfindungsgemäßen Verfahrens legt man den Aldehyd in Gegenwart des Kaliumcarboxylats zusammen mit dem Eisen in einem geeigneten Reaktor, z.B. in einem mit einem Anströmboden versehenen Rohrreaktor, der gegebenenfalls noch Füllkörper enthält, vor und leitet den Sauerstoff oder das Sauerstoff enthaltende Gasgemisch von unten durch den Aldehyd.

Entsprechend einer weiteren Ausführungsform verwendet man als Reaktor einen Rieselturm, der Füllkörper enthält. Über die Füllung läßt man den Kaliumcarboxylat sowie Eisen enthaltenden Aldehyd herabrieseln und leitet in den Turm gleichzeitig im Gleich- oder Gegenstrom, Sauerstoff oder ein Sauerstoff enthaltendes Gasgemisch ein.

In den folgenden Beispielen wird die Herstellung von n-Pentansäure und Isononansäure nach dem beanspruchten Verfahren beschrieben.

Die Umsetzung der Ausgangsaldehyde erfolgt entsprechend der Erfindung in Gegenwart von Kaliumcarboxylat und von Eisen oder Eisenverbindungen als Katalysatoren. Den Beispielen wurden die Ergebnisse von Vergleichsbeispielen gegenübergestellt, in denen die Aldehyde ohne jegliche Zusätze, nur unter Zusatz von Kaliumcarboxylat sowie nur unter Zusatz von Eisen als katalytischem Metall oxidiert wurden.

Die jeweiligen Versuchsergebnisse werden durch die Angaben folgender Kenngrößen wiedergegeben:
- Aldehyd-Umsatz;
- Selektivität, sie ergibt sich aus dem Carbonsäure-Anteil im Reaktionsprodukt, bezogen auf umgesetzten Aldehyd;
- Ausbeute an Carbonsäure

Selbstverständlich ist das neue Verfahren nicht auf die nachstehend beschriebene Ausführungsform beschränkt.

### Beispiele

### Oxidation von n-Pentanal

### Vergleichsbeispiel 1 (ohne Zusatz von Kalium und Eisen):

Die Flüssigphasenoxidation von n-Pentanal zu n-Pentansäure wurde ohne Katalysatorzusatz in einem Blasensäulenreaktor aus Glas mit einem Innendurchmesser von 38 mm und 150 cm Länge durchgeführt. Abhängig vom Reaktionsverhalten wurde der Reaktor mantelseitig durch einen mit einem Wärmetauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40 µm.

In die Oxidation wurde ein Gemisch aus 765,0 g n-Pentanal und 35,0 g n-Pentansäure eingesetzt. Entsprechend der gaschromatographischen Analyse (GC-Analyse) hatte der Aldehyd folgende Zusammensetzung:

| | |
|---|---|
| 0,01 % | Vorlaufkomponenten |
| 0,23 % | 2-/3-Methylbutanal |
| 99,62 % | n-Pentanal |
| 0,11 % | n-Pentanol |
| 0,03 % | n-Pentansäure |

Nach 6 Stunden Oxidation bei konstant 50°C und einem Sauerstoffeinsatz von insgesamt 120 % d.Th., bezogen auf eingesetzten Aldehyd, wurden folgende Ergebnisse ermittelt:

| | | |
|---|---|---|
| GC-Analyse: | 0,20 % | Vorlaufkomponenten |
| | 3,28 % | n-Pentanal |
| | 0,20 % | 2-/3-Methylbuttersäure |
| | 95,42 % | n-Pentansäure |
| | 0,90 % | Sonstige |

Der Umsatz an n-Pentanal beträgt 96,0 % d.Th., die zugehörige Selektivität zur Bildung von n-Pentansäure 99,0 % d.Th..

Daraus lässt sich eine Ausbeute von 95,0 % berechnen. Der im Oxidationseinsatz bereits befindliche Anteil an Carbonsäure wurde bei der Ergebnisberechnung berücksichtigt.

### Vergleichsbeispiel 2 (ohne Zusatz von Eisen):

Dieser Versuch wurde unter den Bedingungen des Vergleichsbeispiels 1 durchgeführt mit der Abweichung, dass dem Einsatzaldehyd (765,0 g) neben n-Pentansäure (29,5 g) auch noch eine Katalysatorlösung enthaltend 2,50 g Kalium-n-pentanoat, 5,47 g n-Pentansäure und 1,32 g Wasser zugesetzt wurde. Das molare Verhältnis n-Pentanal zu Kalium betrug 1000 zu 2.

Nach 6 Stunden Oxidation bei konstant 50°C und einem Sauerstoffeinsatz von insgesamt 120 % d.Th., bezogen auf eingesetzten Aldehyd, wurden folgende Ergebnisse ermittelt:

| | | |
|---|---|---|
| GC-Analyse: | 0,12 % | Vorlaufkomponenten |
| | 3,97 % | n-Pentanal |
| | 0,16 % | 2-/3-Methylbuttersäure |
| | 95,28 % | n-Pentansäure |
| | 0,47 % | Sonstige |

Der Umsatz an n-Pentanal beträgt 95,1 % d.Th., die zugehörige Selektivität zur Bildung von n-Pentansäure 99,6 % d.Th..

Daraus lässt sich eine Ausbeute von 94,7 % berechnen. Der im Oxidationseinsatz bereits befindliche Anteil an Carbonsäure wurde bei der Ergebnisberechnung berücksichtigt.

### Vergleichsbeispiel 3 (ohne Zusatz von Eisen):

Das Vergleichsbeispiel 2 wurde wiederholt mit der Abweichung, dass dem Einsatzaldehyd (765,0 g) neben n-Pentansäure (21,4 g) auch noch eine Katalysatorlösung enthaltend 6,22 g Kalium-n-pentanoat, 13,59 g n-Pentansäure und 3,29 g Wasser zugesetzt wurde. Das molare Verhältnis n-Pentanal zu Kalium betrug 1000 zu 5.

Nach 6 Stunden Oxidation bei konstant 50°C und einem Sauerstoffeinsatz von insgesamt 120 % d.Th., bezogen auf eingesetzten Aldehyd, wurden folgende Ergebnisse ermittelt:

| | | |
|---|---|---|
| GC-Analyse: | 0,11 % | Vorlaufkomponenten |
| | 4,76 % | n-Pentanal |
| | 0,20 % | 2-/3-Methylbuttersäure |
| | 94,49 % | n-Pentansäure |
| | 0,44 % | Sonstige |

Der Umsatz an n-Pentanal beträgt 94,2 % d.Th., die zugehörige Selektivität zur Bildung von n-Pentansäure 99,6 % d.Th..

Daraus lässt sich eine Ausbeute von 93,8 % berechnen. Der im Oxidationseinsatz bereits befindliche Anteil an Carbonsäure wurde bei der Ergebnisberechnung berücksichtigt.

### Vergleichsbeispiel 4 (ohne Zusatz von Kalium):

Dieser Versuch wurde unter den Bedingungen des Vergleichsbeispiels 1 durchgeführt mit der Abweichung, dass dem Einsatzaldehyd (765,0 g) neben n-Pentansäure (25,0 g) auch noch 10,0 g n-Pentansäure mit einem Gehalt von 0,49 mg Fe als Katalysatorlösung zugesetzt wurden. Bezogen auf den Aldehyd betrug der Eisenzusatz 0,63 ppm.

Nach 6 Stunden Oxidation bei konstant 50°C und einem Sauerstoffeinsatz von insgesamt 120 % d.Th., bezogen auf eingesetzten Aldehyd, wurden folgende Ergebnisse ermittelt:

| | | |
|---|---|---|
| GC-Analyse: | 0,47 % | Vorlaufkomponenten |
| | 0,82 % | n-Pentanal |
| | 0,20 % | 2-/3-Methylbuttersäure |
| | 97,22 % | n-Pentansäure |
| | 1,29 % | Sonstige |

Der Umsatz an n-Pentanal beträgt 99,0 % d.Th., die zugehörige Selektivität zur Bildung von n-Pentansäure 98,3 % d.Th..

Daraus lässt sich eine Ausbeute von 97,3 % berechnen. Der im Oxidationseinsatz bereits befindliche Anteil an Carbonsäure wurde bei der Ergebnisberechnung berücksichtigt.

### Vergleichsbeispiel 5 (ohne Zusatz von Kalium):

Das Vergleichsbeispiel 4 wurde wiederholt mit der Abweichung, dass dem Einsatzaldehyd (765,0 g) neben n-Pentansäure (15,0 g) auch noch 20,0 g n-Pentansäure mit einem Gehalt von 0,97 mg Fe als Katalysatorlösung zugesetzt wurden. Bezogen auf den Aldehyd betrug der Eisenzusatz 1,27 ppm.

Nach 6 Stunden Oxidation bei konstant 50°C und einem Sauerstoffeinsatz von insgesamt 120 % d.Th., bezogen auf eingesetzten Aldehyd, wurden folgende Ergebnisse ermittelt:

| | | |
|---|---|---|
| GC-Analyse: | 0,49 % | Vorlaufkomponenten |
| | 0,63 % | n-Pentanal |
| | 0,21 % | 2-/3-Methylbuttersäure |
| | 96,89 % | n-Pentansäure |
| | 1,78 % | Sonstige |

Der Umsatz an n-Pentanal beträgt 99,2 % d.Th., die zugehörige Selektivität zur Bildung von n-Pentansäure 97,8 % d.Th..

Daraus lässt sich eine Ausbeute von 97,0 % berechnen. Der im Oxidationseinsatz bereits befindliche Anteil an Carbonsäure wurde bei der Ergebnisberechnung berücksichtigt.

### Beispiel 1

Dieser Versuch wurde unter den Bedingungen des Vergleichsbeispiels 1 durchgeführt mit der Abweichung, dass dem Einsatzaldehyd (765,0 g) neben n-Pentansäure (19,5 g) auch noch zwei Katalysatorlösungen zugesetzt wurden. Die Katalysatorlösung A enthielt 2,50 g Kalium-n-pentanoat, 5,47 g n-Pentansäure und 1,32 g Wasser, als Katalysatorlösung B wurden 10,0 g n-Pentansäure mit einem Gehalt von 0,49 mg Fe eingesetzt.
Das molare Verhältnis n-Pentanal zu Kalium betrug damit 1000 zu 2, bezogen auf den Aldehyd betrug der Eisenzusatz 0,63 ppm.

Nach 6 Stunden Oxidation bei konstant 50°C und einem Sauerstoffeinsatz von insgesamt 120 % d.Th., bezogen auf eingesetzten Aldehyd, wurden folgende Ergebnisse ermittelt:

| | | |
|---|---|---|
| GC-Analyse: | 0,16 % | Vorlaufkomponenten |
| | 0,74 % | n-Pentanal |
| | 0,21 % | 2-/3-Methylbuttersäure |
| | 98,22 % | n-Pentansäure |
| | 0,67 % | Sonstige |

Der Umsatz an n-Pentanal beträgt 99,1 % d.Th., die zugehörige Selektivität zur Bildung von n-Pentansäure 99,3 % d.Th..

Daraus lässt sich eine Ausbeute von 98,4 % berechnen. Der im Oxidationseinsatz bereits befindliche Anteil an Carbonsäure wurde bei der Ergebnisberechnung berücksichtigt.

### Beispiele 2-4

Die Beispiele 2 bis 4 wurden unter den Bedingungen des Beispiels 1 durchgeführt mit der Abweichung, dass dem Einsatzaldehyd (765,0 g) unterschiedliche Mengen an Katalysatorlösung A und B zugesetzt wurden. Weitere Einzelheiten sind der Tabelle 1 zu entnehmen.

**Tabelle 1: Oxidation von n-Pentanal in Gegenwart von Kalium und Eisen**

| Versuch | | 2 | 3 | 4 |
|---|---|---|---|---|
| Einsatzmenge n-Pentanal (g) | | 765,0 | 765,0 | 765,0 |
| Einsatzmenge Katalysatorlösung A (g) | | 9,3 | 23,1 | 23,1 |
| Einsatzmenge Katalysatorlösung B (g) | | 20,0 | 10,0 | 20,0 |
| | | | | |
| Zusammensetzung der Katalysatorlösung A | | | | |
| | - Kalium-n-pentanoat (g) | 2,50 | 6,22 | 6,22 |
| | - n-Pentansäure (g) | 5,47 | 13,59 | 13,59 |
| | - Wasser (g) | 1,32 | 3,29 | 3,29 |
| Zusammensetzung der Katalysatorlösung B | | | | |
| | - n-Pentansäure (g) | 20,0 | 10,0 | 20,0 |
| | - Eisen (mg) | 0,97 | 0,49 | 0,97 |
| Molares Verhältnis n-Pentanal zu Kalium | | 1000 zu 2 | 1000 zu 5 | 1000 zu 5 |
| Eisenzusatz bez. auf n-Pentanal (ppm) | | 1,27 | 0,63 | 1,27 |
| GC-Analyse der Rohsäure | | | | |
| | - Vorlaufkomponenten (%) | 0,17 | 0,16 | 0,16 |
| | - n-Pentanal (%) | 0,61 | 0,88 | 0,60 |
| | - 2-/3-Methylbuttersäure (%) | 0,21 | 0,21 | 0,22 |
| | - n-Pentansäure (%) | 98,23 | 98,15 | 98,38 |
| | - Sonstige (%) | 0,78 | 0,60 | 0,64 |
| Umsatz n-Pentanal (% d.Th.) | | 99,2 | 98,9 | 99,3 |
| Selektivität n-Pentansäure (% d.Th.) | | 99,2 | 99,4 | 99,3 |
| Ausbeute n-Pentansäure (% d.Th.) | | 98,4 | 98,3 | 98,6 |

### Oxidation von Isononanal

### Vergleichsbeispiel 6 (ohne Zusatz von Kalium und Eisen):

Die Flüssigphasenoxidation von Isononanal zu Isononansäure wurde ohne Katalysatorzusatz in einem Blasensäulenreaktor aus Glas mit einem Innendurchmesser von 38 mm und 150 cm Länge durchgeführt. Abhängig vom Reaktionsverhalten wurde der Reaktor mantelseitig durch einen mit einem Wärmetauscher verbundenen Wasserkreislauf gekühlt oder beheizt und die Innentemperatur auf diese Weise konstant gehalten. Die Sauerstoffzufuhr erfolgte von unten durch eine mit der Blasensäule verbundene Glasfilterplatte mit einer maximalen Porenweite von 16-40 µm.

In die Oxidation wurde ein Gemisch aus 700,0 g Isononanal und 100,0 g Isononansäure eingesetzt. Entsprechend der gaschromatographischen Analyse hatte der Aldehyd folgende Zusammensetzung:

| | |
|---|---|
| 0,16 % | Vorlaufkomponenten |
| 94,41 % | 3,5,5-Trimethylhexanal |
| 5,24 % | isomere C₉-Aldehyde |
| 0,04 % | 3,5,5-Trimethylhexansäure |
| 0,15 % | Sonstige |

Nach 6 Stunden Oxidation bei konstant 60°C und einem Durchsatz von 20 Liter Sauerstoff/Stunde wurden folgende Ergebnisse ermittelt:

| | | |
|---|---|---|
| GC-Analyse: | 0,58 % | Vorlaufkomponenten |
| | 1,74 % | 3,5,5-Trimethylhexanal |
| | 0,07 % | isomere C₉-Aldehyde |
| | 90,81 % | 3,5,5-Trimethylhexansäure |
| | 5,35 % | isomere C₉-Säuren |
| | 1,45 % | Sonstige |

Der Umsatz (bezogen auf die Hauptkomponente 3,5,5-Trimethylhexanal) beträgt 97,7 % d.Th., die zugehörige Selektivität zur Bildung von 3,5,5-Trimethylhexansäure 97,7 % d.Th..

Daraus lässt sich eine Ausbeute von 95,5 % berechnen. Der im Oxidationseinsatz bereits befindliche Anteil an Carbonsäure wurde bei der Ergebnisberechnung berücksichtigt.

### Vergleichsbeispiel 7 (ohne Zusatz von Eisen):

Dieser Versuch wurde unter den Bedingungen des Vergleichsbeispiels 6 durchgeführt mit der Abweichung, dass dem Einsatzaldehyd (700,0 g) neben Isononansäure (88,3 g) auch noch eine Katalysatorlösung enthaltend 4,82 g Kalium-isononanoat, 11,66 g Isononansäure und 1,82 g Wasser zugesetzt wurde. Das molare Verhältnis n-Pentanal zu Kalium betrug 1000 zu 5.

Nach 6 Stunden Oxidation bei konstant 60°C und einem Durchsatz von 20 Liter Sauerstoff/Stunde wurden folgende Ergebnisse ermittelt:

| | | |
|---|---|---|
| GC-Analyse: | 0,28 % | Vorlaufkomponenten |
| | 0,97 % | 3,5,5-Trimethylhexanal |
| | 0,10 % | isomere C₉-Aldehyde |
| | 92,54 % | 3,5,5-Trimethylhexansäure |
| | 5,69 % | isomere C₉-Säuren |
| | 0,42 % | Sonstige |

Der Umsatz (bezogen auf die Hauptkomponente 3,5,5-Trimethylhexanal) beträgt 98,7 % d.Th., die zugehörige Selektivität zur Bildung von 3,5,5-Trimethylhexansäure 98,9 % d.Th..

Daraus lässt sich eine Ausbeute von 97,6 % berechnen. Der im Oxidationseinsatz bereits befindliche Anteil an Carbonsäure wurde bei der Ergebnisberechnung berücksichtigt.

### Vergleichsbeispiel 8 (ohne Zusatz von Kalium):

Dieser Versuch wurde unter den Bedingungen des Vergleichsbeispiels 6 durchgeführt mit der Abweichung, dass dem Einsatzaldehyd (700,0 g) neben Isononansäure (11,7 g) auch noch 88,3 g Isononansäure mit einem Gehalt von 0,51 mg Fe als Katalysatorlösung zugesetzt wurden. Bezogen auf den Aldehyd betrug der Eisenzusatz 0,73 ppm.

Nach 6 Stunden Oxidation bei konstant 60°C und einem Durchsatz von 20 Liter Sauerstoff/Stunde wurden folgende Ergebnisse ermittelt:

| | | |
|---|---|---|
| GC-Analyse: | 0,95 % | Vorlaufkomponenten |
| | 0,56 % | 3,5,5-Trimethylhexanal |
| | 0,13 % | isomere C₉-Aldehyde |
| | 91,46 % | 3,5,5-Trimethylhexansäure |
| | 5,37 % | isomere C₉-Säuren |
| | 1,53 % | Sonstige |

Der Umsatz (bezogen auf die Hauptkomponente 3,5,5-Trimethylhexanal) beträgt 99,3 % d.Th., die zugehörige Selektivität zur Bildung von 3,5,5-Trimethylhexansäure 97,1 % d.Th..

Daraus lässt sich eine Ausbeute von 96,4 % berechnen. Der im Oxidationseinsatz bereits befindliche Anteil an Carbonsäure wurde bei der Ergebnisberechnung berücksichtigt.

### Beispiel 5

Dieser Versuch wurde unter den Bedingungen des Vergleichsbeispiels 6 durchgeführt mit der Abweichung, dass dem Einsatzaldehyd (700,0 g) auch noch zwei Katalysatorlösungen zugesetzt wurden. Die Katalysatorlösung A enthielt 4,82 g Kalium-isononanoat, 11,66 g Isononansäure und 1,82 g Wasser, als Katalysatorlösung B wurden 88,3 g Isononansäure mit einem Gehalt von 0,51 mg Fe eingesetzt.

Das molare Verhältnis Isononanal zu Kalium betrug 1000 zu 5, bezogen auf den Aldehyd betrug der Eisenzusatz 0,73 ppm.

Nach 6 Stunden Oxidation bei konstant 60°C und einem Durchsatz von 20 Liter Sauerstoff/Stunde wurden folgende Ergebnisse ermittelt:

| | | |
|---|---|---|
| GC-Analyse: | 0,38 % | Vorlaufkomponenten |
| | 0,22 % | 3,5,5-Trimethylhexanal |
| | 0,08 % | isomere C₉-Aldehyde |
| | 93,21 % | 3,5,5-Trimethylhexansäure |
| | 5,66 % | isomere C₉-Säuren |
| | 0,45 % | Sonstige |

Der Umsatz (bezogen auf die Hauptkomponente 3,5,5-Trimethylhexanal) beträgt 99,7 % d.Th., die zugehörige Selektivität zur Bildung von 3,5,5-Trimethylhexansäure 98,8 % d.Th..

Daraus lässt sich eine Ausbeute von 98,5 % berechnen. Der im Oxidationseinsatz bereits befindliche Anteil an Carbonsäure wurde bei der Ergebnisberechnung berücksichtigt.

In den nachfolgenden Tabellen 2 und 3 sind die Ergebnisse der Vergleichsbeispiele und die Ergebnisse der erfindungsgemäßen Beispiele zusammengefaßt.

**Tabelle 2: Oxidation von n-Pentanal**

| Versuch | Kaliumzusatz [mmol Kalium] je mol Aldehyd | Eisenzusatz [ppm Eisen] | n-Pentanal Umsatz % | Selektivität zu n-Pentansäure % | Ausbeute an n-Pentansäure % |
|---|---|---|---|---|---|
| Vergleich 1 | ohne | ohne | 96,0 | 99,0 | 95,0 |
| Vergleich 2 | 2 | ohne | 95,1 | 99,6 | 94,7 |
| Vergleich 3 | 5 | ohne | 94,2 | 99,6 | 93,8 |
| Vergleich 4 | ohne | 0,63 | 99,0 | 98,3 | 97,3 |
| Vergleich 5 | ohne | 1,27 | 99,2 | 97,8 | 97,0 |
| Beispiel 1 | 2 | 0,63 | 99,1 | 99,3 | 98,4 |
| Beispiel 2 | 2 | 1,27 | 99,2 | 99,2 | 98,4 |
| Beispiel 3 | 5 | 0,63 | 98,9 | 99,4 | 98,3 |
| Beispiel 4 | 5 | 1,27 | 99,3 | 99,3 | 98,6 |

**Tabelle 3: Oxidation von Isononanal**

| Versuch | Kaliumzusatz [mmol Kalium] je mol Aldehyd | Eisenzusatz [ppm Eisen] | Isononanal Umsatz % | Selektivität zu Isononansäure % | Ausbeute an Isononansäure % |
|---|---|---|---|---|---|
| Vergleich 6 | ohne | ohne | 97,7 | 97,7 | 95,5 |
| Vergleich 7 | 5 | ohne | 98,7 | 98,9 | 97,6 |
| Vergleich 8 | ohne | 0,73 | 99,3 | 97,1 | 96,4 |
| Beispiel 5 | 5 | 0,73 | 99,7 | 98,8 | 98,5 |

Wie ein Vergleich der Beispiele belegt, läßt sich das Umsatzverhalten in der Oxidation von aliphatischen geradkettigen oder β-alkylverzweigten Aldehyden zu den entsprechenden Carbonsäuren bei gleichzeitiger Erhöhung der Selektivität verbessern, wenn die Oxidation in Gegenwart von Kaliumcarboxylaten und Eisen als katalytisch aktivem Metall durchgeführt wird.

## Patentansprüche

1. Verfahren zur Herstellung von aliphatischen geradkettigen und von βalkylverzweigten Carbonsäuren mit 5 bis 13 Kohlenstoffatomen durch Oxidation der entsprechenden Aldehyde mit Sauerstoff oder Sauerstoff enthaltenden Gasgemischen bei 20 bis 100°C, **dadurch gekennzeichnet, dass** die Oxidation der Aldehyde in Gegenwart von Kaliumcarboxylat in einer Menge, berechnet als Kaliummetall, von 1 mmol bis 10 mmol je mol eingesetztem Aldehyd und in Gegenwart von 0,1 bis 5,0 ppm Eisen oder der entsprechenden Menge einer Eisenverbindung, bezogen auf eingesetzten Aldehyd, erfolgt.

2. Verfahren nach Anspruch 1, **dadurch gekennzeichnet, dass** die Oxidation der Aldehyde in Gegenwart von Kaliumcarboxylat in einer Menge, berechnet als Kaliummetall, von 1 bis 8 mmol, insbesondere von 1 bis 5 mmol, je mol eingesetztem Aldehyd und in Gegenwart von 0,2 bis 3 ppm, insbesondere von 0,5 bis 2 ppm Eisen oder der entsprechenden Menge einer Eisenverbindung, bezogen auf eingesetzten Aldehyd, erfolgt.

3. Verfahren nach Anspruch 1 oder 2, **dadurch gekennzeichnet, dass** die Kaliumcarboxylate Salze der Carbonsäuren sind, die als Ergebnis der Oxidation der eingesetzten Aldehyde entstehen.

4. Verfahren nach einem oder mehreren der Ansprüche 1 bis 3, **dadurch gekennzeichnet, dass** die Eisenverbindungen Carboxylate, Acetylacetonate oder Carbonylverbindungen sind.

5. Verfahren nach Anspruch 4, **dadurch gekennzeichnet, dass** die Eisencarboxylate Salze der Carbonsäuren sind, die als Ergebnis der Oxidation der eingesetzten Aldehyde entstehen.

6. Verfahren nach einem oder mehreren der Ansprüche 1 bis 5, **dadurch gekennzeichnet, dass** die Oxidation bei Temperaturen im Bereich von 20 bis 80°C, vorzugsweise 40 bis 80°C, durchgeführt wird.

7. Verfahren nach einem oder mehreren der Ansprüche 1 bis 6, **dadurch gekennzeichnet, dass** die Oxidation bei Drücken in einem Bereich von Atmosphärendruck bis 1,0 MPa, vorzugsweise Atmosphärendruck bis 0,8 MPa durchgeführt wird.

8. Verfahren nach einem oder mehreren der Ansprüche 1 bis 7, **dadurch gekennzeichnet, dass** die Sauerstoff enthaltenden Gasgemische einen Anteil von bis zu 90 Vol.-%, insbesondere 30 bis 80 Vol.-%, inerter Bestandteile aufweisen.

## Claims

1. A process for preparing aliphatic straight-chain and β-alkyl-branched carboxylic acids having from 5 to 13 carbon atoms by oxidation of the corresponding aldehydes by means of oxygen or oxygen-containing gas mixtures at from 20 to 100°C, wherein the oxidation of the aldehydes is carried out in the presence of potassium carboxylate in an amount, calculated as potassium metal, of from 1 mmol to 10 mmol per mole of aldehyde used and in the presence of from 0.1 to 5.0 ppm of iron or the corresponding amount of an iron compound, based on the aldehyde used.

2. The process as claimed in claim 1, wherein the oxidation of the aldehydes is carried out in the presence of potassium carboxylate in an amount, calculated as potassium metal, of from 1 mmol to 8 mmol, in particular from 1 mmol to 5 mmol, per mole of aldehyde used and in the presence of from 0.2 to 3 ppm, in particular from 0.5 to 2 ppm, of iron or the corresponding amount of an iron compound, based on the aldehyde used.

3. The process as claimed in claim 1 or 2, wherein the potassium carboxylates are salts of the carboxylic acids which are formed as a result of the oxidation of the aldehydes used.

4. The process as claimed in one or more of claims 1 to 3, wherein the iron compounds are carboxylates, acetylacetonates or carbonyl compounds.

5. The process as claimed in claim 4, wherein the iron carboxylates are salts of the carboxylic acids which are formed as a result of the oxidation of the aldehydes used.

6. The process as claimed in one or more of claims 1 to 5, wherein the oxidation is carried out at temperatures in the range from 20 to 80°C, preferably from 40 to 80°C.

7. The process as claimed in one or more of claims 1 to 6, wherein the oxidation is carried out at pressures in a range from atmospheric pressure to 1.0 MPa, preferably from atmospheric pressure to 0.8 MPa.

8. The process as claimed in one or more of claims 1 to 7, wherein the oxygen-containing gas mixtures have a proportion of up to 90% by volume, in particular from 30 to 80% by volume, of inert constituents.

## Revendications

1. Procédé pour la préparation d'acides carboxyliques aliphatiques linéaires et ramifiés par alkyle en position β comprenant 5 à 13 atomes de carbone par oxydation des aldéhydes correspondants avec de l'oxygène ou des mélanges gazeux contenant de l'oxygène à 20 jusqu'à 100°C, **caractérisé en ce que** l'oxydation des aldéhydes est réalisée en présence de carboxylate de potassium en une quantité, calculée sous forme de potassium métallique, de 1 mmole à 10 mmoles par mole d'aldéhyde utilisé et en présence de 0,1 à 5,0 ppm de fer ou de la quantité correspondante d'un composé à base de fer, par rapport à l'aldéhyde utilisé.

2. Procédé selon la revendication 1, **caractérisé en ce que** l'oxydation des aldéhydes est réalisée en présence de carboxylate de potassium en une quantité, calculée sous forme de potassium métallique, de 1 à 8 mmoles, en particulier de 1 à 5 mmoles, par mole d'aldéhyde utilisé et en présence de 0,2 à 3 ppm, en particulier de 0,5 à 2 ppm de fer ou de la quantité correspondante d'un composé à base de fer, par rapport à l'aldéhyde utilisé.

3. Procédé selon la revendication 1 ou 2, **caractérisé en ce que** les carboxylates de potassium sont des sels des acides carboxyliques qui sont formés comme résultat de l'oxydation des aldéhydes utilisés.

4. Procédé selon l'une ou plusieurs des revendications 1 à 3, **caractérisé en ce que** les composés du fer sont des carboxylates, des acétylacétonates ou des composés de type carbonyle.

5. Procédé selon la revendication 4, **caractérisé en ce** les carboxylates de fer sont des sels des acides carboxyliques qui sont formés comme résultat de l'oxydation des aldéhydes utilisés.

6. Procédé selon l'une ou plusieurs des revendications 1 à 5, **caractérisé en ce que** l'oxydation est réalisée à des températures dans la plage de 20 à 80°C, de préférence de 40 à 80°C.

7. Procédé selon l'une ou plusieurs des revendications 1 à 6, **caractérisé en ce que** l'oxydation est réalisée à des pressions dans une plage allant de la pression atmosphérique jusqu'à 1,0 MPa, de préférence de la pression atmosphérique jusqu'à 0,8 MPa.

8. Procédé selon l'une ou plusieurs des revendications 1 à 7, **caractérisé en ce que** les mélanges gazeux contenant l'oxygène présentent une proportion allant jusqu'à 90% en volume, en particulier de 30 à 80% en volume, de constituants inertes.
